# EUROPEAN PATENT APPLICATION

(11) **EP 1 435 229 A1**
(43) Date of publication of application: **07.07.2004**
(21) Application number: 03012320.2
(22) Date of filing: 29.05.2003
(51) Int. Cl.: A61K 7/48, A61K 7/16

(54) **Dermatologic composition for topical-use dermatologic products and dermatologic products made thereby**

(30) Priority: 30.12.2002 IT MI20022781
(71) Applicant: Farmaceutici S.R.L., 15057 Tortona, Alessandria (IT)
(72) Inventor: Bonabello, Elvio, 15067 Novi Ligure (AL) (IT)
(74) Representative: Cicogna, Franco

(57) **Abstract**

A dermatologic composition for topical-use dermatologic products comprises, as a specific characteristic that of using, as a matrix and solvent therefor, isotonic and/or hypertonic sea water, depending on the requirements considered as ideal for each individual product.

With respect to conventional topical-use dermatologic products, the products prepared by the dermatologic composition according to the present invention provide the advantage of fully exploiting the effect of the active principles thereof, owing to the extraordinary conveying properties of the sea water towards the minerals dissolved therein.

## Description

### BACKGROUND OF THE INVENTION

The present invention relates to a novel dermatologic composition for topical-use dermatologic products, and also relates to dermatologic products made thereby.

As is known, topical-use dermatologic products are prepared starting from dermatologic compositions which conventionally comprise an aqueous base, which, at present, is constituted by denaturated water.

However, conventional denaturated water bases for the above mentioned applications, have the drawback that they are not suitable to sufficiently convey the mineral elements dissolved therein, which would make transcutaneous absorption very difficult or limited.

### SUMMARY OF THE INVENTION

Accordingly, the aim of the present invention is to provide a novel dermatologic composition for topical-use dermatologic products, which, with respect to conventional denaturated water based like dermatologic composition, provides an improved transcutaneous absorption of the active principles thereof, independently from their cleansing, anti-inflammatory, disinfectant or cosmetic properties.

Within the scope of the above mentioned aim, a main object of the present invention is to provide novel topical-use dermatologic products which are more efficient than like prior dermatologic products.

According to one aspect of the present invention, the above mentioned aim and objects, as well as yet other objects, which will become more apparent hereinafter, are achieved with the dermatologic composition and dermatologic products according to claims 1 and 7, respectively.

Further embodiments of the invention are defined in the dependent claims.

With respect to conventional topical-use dermatologic products, the dermatologic products prepared with the dermatologic composition according to the present invention provide the advantage that they are able to fully exploit the dermatologic effect of the active principles thereof, owing to the extraordinary conveying properties of the sea water towards the minerals dissolved therein.

In particular, the dermatologic composition for topical-use dermatologic products according to the present invention has, as a specific characteristic thereof, that of using, as a matrix and solvent therefor, isotonic and/or ipertonic sea water, according to the requirements considered as ideal for each intended individual product.

The interstitial liquid, present between the connective cells, has characteristics absolutely similar to those of sea water, which, accordingly, not only is electively indicated as a solvent, but also as a conveying or carrying material, since the mineral elements dissolved therein make it more permeable to the protective lipidic layer of the skin, thereby enhancing the active principle action.

The sea water based dermatologic composition according to the present invention, is accordingly very suitable for association with disinfecting, anti-inflammatory, nutrient, cosmetic and hydrating substances, for topic use, in the form of dermatologic products, for topical application, having cleansing, cosmetic, anti-inflammatory, draining, anti-redding, conveying and like properties.

Thus, the used sea water can be suitably diluted and made isotonic for long period treatments, but it can also be used in a pure condition, or with intermediate dilutions, for treatments in which it is considered more suitable.

Hereinbelow will be indicated some examples of topical-use dermatologic product formulations, prepared with the sea water based composition according to the present invention.

The above mentioned products can be in the form of creams, emulsions, aqueous solutions, alkaline solutions, oil solutions, gels, cream-gels and ointments.

They are suitable for application to any desired portion of human body, such as the skin, hair, hairs and nails.

### EXAMPLE 1

50 ml of a cosmetic product have been prepared starting from 50 ml of sea water taken from a marine park and processed as follows: after sterilization by gamma process, it has been diluted with 450 ml of denatured water and then brought to an isotonic level with the body liquids.

The following active principles have been then added, in the below indicated rates:
- Arnica 11%
- Hamamelis Virginiana water 3%
- Sea collagen 2%
- Ruscus 2.5%
- Pure Escine 1%
- Polyunsaturated fat acids 1%

Since this formulation corresponds to the same formulation of a first cosmetic product having a similar composition, but emulsified with normal denaturated water, a comparative experimentation has been carried out.

The faces of five women have been treated for 15 days with the similar product with denaturated water and, after a break of 30 days, the treatment has been repeated by using the second cosmetic product in sea water, for an equal period of time.

All the five women have declared that they noticed, with the second treatment, greater skin hydratation, improved feeling of freshness of the face, and greater softness and smoothness of the skin.

### EXAMPLE 2

To 50 ml of an isotonic and steril sea water solution, some natural active principles, as those conventionally used in the phitopharmatologic field have been added.

The active principles and the rates thereof were:
- Beta-glicirretic acid 18: 0.75 grams
- Allantoine: 0.50 grams
- Escine: 1 gram

The composition has been used for 10 days on a pedriatic age patient (8 years) affected by atopic dermatitis, with the following results: after only 5 days, the affected skin surface decreased by 50%, and the degree of redding of the remaining part was merely recover at an advance stage of; on the tenth day the skin rash had fully disappeared.

The subject under examination had already been treated with other analogous products, but the treatment had to be suspended since it causes an allergic reaction.

On the contrary, by using the composition according to the invention, the patient was not affected by any allergic reaction through out the overall treatment period.

Sea water can be used in cosmetic compositions such as tooth pastes and/or mouth washes, for which it would provide a synergic action with the substances constituting normal tooth pastes or cosmetic compositions.

## Claims

1. A water based composition for preparing topical use dermatologic products, **characterized in that** said water base comprises sea water containing natural and/or synthetic active principles.

2. A composition according to Claim 1,
**characterized in that** said sea water is isotonic and/or hypertonic sea water.

3. A composition according to Claim 1 or 2, **characterized in that** said composition comprises one or more disinfectant substances.

4. A composition according to Claim 1 or 2, **characterized in that** said composition comprises one or more anti-inflammatory substances.

5. A composition according to Claim 1 or 2, **characterized in that** said composition comprises one or more cleansing substances.

6. A composition according to Claim 1 or 2, **characterized in that** said composition comprises one or more emulsifying, surface active and/or carrier substances.

7. A topical use dermatologic product, **characterized in that** said product is prepared with a composition according to one or more of the preceding claims.

8. A product according to Claim 7,
**characterized in that** said product is a disinfectant products.

9. A product according to Claim 7,
**characterized in that** said product is an anti-inflammatory product.

10. A product according to Claim 7,
**characterized in that** said product is a cleansing product.

11. A product according to one or more of Claims 7 to 10, **characterized in that** said product is in a cream, emulsion, aqueous solution, alkali solution, oil solution, gel, cream gel or ointment form.

12. A product according to one or more of the preceding claims, **characterized in that** said sea water is used in cosmetic products such as tooth pastes or mouth washes.

13. Use of a composition according to Claims 1 and 2 for preparing topical use dermatologic products, substantially as broadly disclosed.
